(19) Europäisches Patentamt
European Patent Office
Office européen des brevets

(11) EP 0 684 257 B1

(12) **FASCICULE DE BREVET EUROPEEN**

(45) Date de publication et mention de la délivrance du brevet:
**22.09.1999 Bulletin 1999/38**

(51) Int. Cl.[6]: **C07K 5/023**, A61K 38/06

(21) Numéro de dépôt: **95400953.6**

(22) Date de dépôt: **27.04.1995**

(54) **Pseudopeptides dérivés de neurokinines, leur procédé de préparation et les compositions pharmaceutiques qui les contiennent**

Pseudopeptidderivate der Neurokinine, Verfahren zu ihrer Herstellung und sie enthaltende pharmazeutische Zusammensetzungen

Pseudopeptide derivatives of neurokinines, process for their preparation and pharmaceutical compositions comprising them

(84) Etats contractants désignés:
**AT BE CH DE DK ES FR GB GR IE IT LI LU NL PT SE**

(30) Priorité: **28.04.1994 FR 9405137**

(43) Date de publication de la demande:
**29.11.1995 Bulletin 1995/48**

(73) Titulaire: **ADIR ET COMPAGNIE**
**92415 Courbevoie Cédex (FR)**

(72) Inventeurs:
- **Fauchere, Jean-Luc**
**F-92210 Saint Cloud (FR)**
- **Kucharczyk-Gentric, Nathalie**
**F-92130 Issy les Moulineaux (FR)**
- **Paladino, Joseph**
**F-78700 Conflans Sainte Honorine (FR)**
- **Bonnet, Jacqueline**
**F-75013 Paris (FR)**
- **Canet, Emmanuel**
**F-75005 Paris (FR)**
- **Birrell, Graham**
**Edinburgh EH10 4JU (GB)**

(56) Documents cités:
**EP-A- 0 443 132**
**WO-A-92/22569**
**WO-A-94/05693**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 684 257 B1

Printed by Xerox (UK) Business Services
2.16.7/3.6

## Description

La présente invention concerne de nouveaux pseudopeptides dérivés de neurokinines, leur procédé de préparation et les compositions pharmaceutiques qui les contiennent.

Les neurokinines forment une famille de neuropeptides possédant à la partie C terminale une analogie de structure : Phe-X-Gly-Leu-Met. Ces neuropeptides, substance (SP), neurokinine (NKA) et neurokinine (NKB) induisent une contraction rapide des fibres musculaires lisses par opposition aux contractions lentes développées par la bradykinine. Largement représentées au niveau du corps humain, en particulier au niveau du système nerveux central et du système nerveux périphérique, leurs effets agonistes endogènes s'effectuent via des récepteurs spécifiques avec une affinité préférentielle pour $NK_1$, $NK_2$, $NK_3$ respectivement pour SP, NKA et NKB. Ils sont impliqués dans de nombreux processus physiologiques ou physiopathologiques tels que nociception, vasoperméabilité, contractions des fibres musculaires lisses, hypersécrétions, et immuno-modulations (OTSUKA M. et coll., Physiol. Rev. 73, 229-308, 1993).
De nombreux peptides antagonistes des neurokinines ont été décrits dans la littérature. C'est le cas par exemple des composés décrits dans les brevets EP 333174, EP 394989 WO 9222569, WO 94 05693 ou EP 443 132.

La présente invention a pour objet des pseudopeptides synthétiques, qui, outre le fait qu'ils soient nouveaux, se sont montrés particulièrement intéressants par l'intensité de leurs propriétés pharmacologiques. Ils possèdent des propriétés antagonistes sélectives et puissantes vis-à-vis des récepteurs des neurokinines et plus particulièrement des récepteurs $NK_1$. Ces propriétés les rendent utilisables notamment dans le traitement de la douleur, des processus inflammatoires d'origines diverses, des troubles gastro-intestinaux, de l'asthme, des allergies, des troubles urologiques, de la migraine et des maladies du système nerveux central.

L'invention concerne plus particulièrement de nouveaux composés de formule (I) :

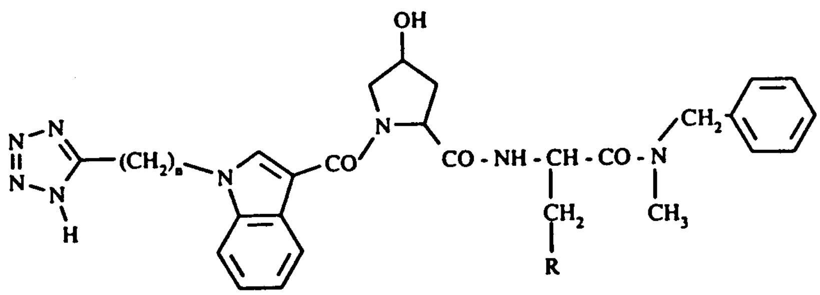

(I)

dans laquelle :

- R représente un groupement naphtyle, 5,6,7,8-tétrahydronaphtyle, 1,2,3,4-tétrahydronaphtyle, thiényle, phényle (éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle) ou cycloalkyle ($C_3$-$C_7$),
- n représente un entier tel que $1 \leq n \leq 8$,

leurs énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend également au procédé de préparation des composés de formule (I) caractérisé en ce que l'on fait réagir une hydroxyproline protégée, de formule (II), dont on a éventuellement séparé les isomères :

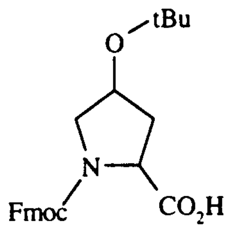

(II)

dans laquelle :

Fmoc représente le groupement 9-fluorénylméthoxycarbonyle,
tBu représente le groupement tert-butyle,
avec de l'alcool benzylique, en présence de 4-diméthylaminopyridine et de N,N-dicyclohexylcarbodiimide, pour conduire au composé de formule (III) :

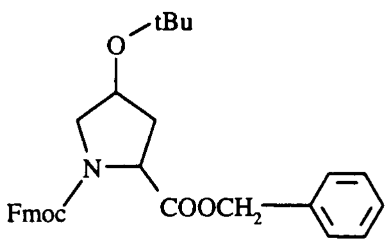

(III)

dans laquelle Fmoc et tBu ont les mêmes significations que précédemment,
dont on déprotège la fonction amine en milieu pipéridine,
pour conduire au composé de formule (IV) :

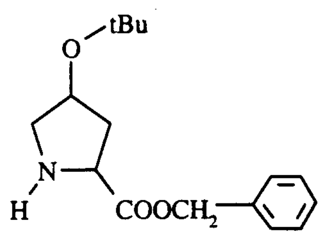

(IV)

dans laquelle tBu a la même signification que précédemment,
que l'on fait réagir avec de l'acide indole-3-carboxylique,
pour conduire au composé de formule (V) :

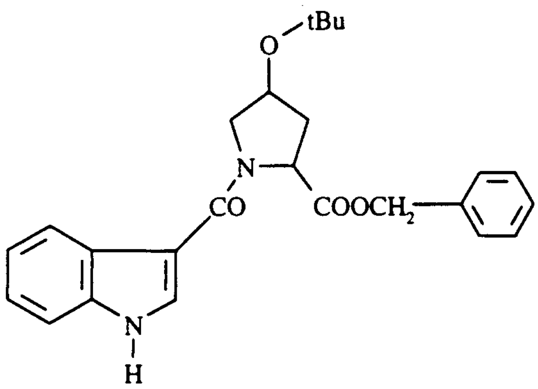

(V)

dans laquelle tBu a la même signification que précédemment, dont on déprotège la fonction acide carboxylique, par hydrogénation catalytique, pour conduire au composé de formule (VI) :

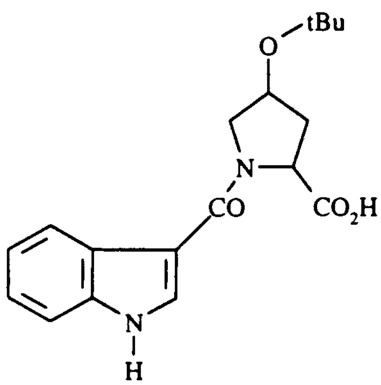

(VI)

dans laquelle tBu a la même signification que précédemment, que l'on met en réaction avec un amino-acide protégé de formule (VII), dont on a éventuellement séparé les isomères :

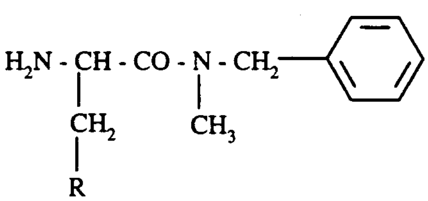

(VII)

dans laquelle R a la même signification que dans la formule (I), pour conduire au composé de formule (VIII) :

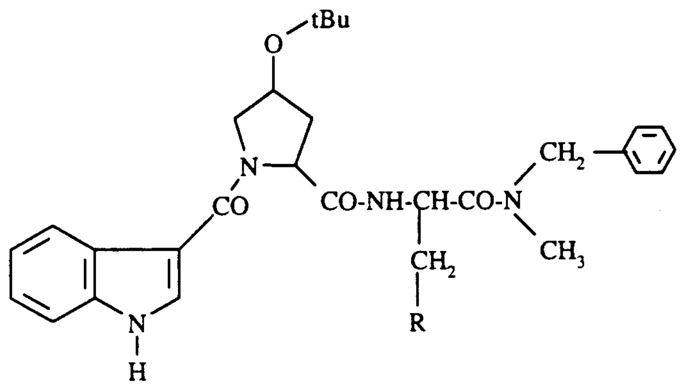

(VIII)

dans laquelle tBu et R ont la même signification que précédemment, que l'on fait réagir sur un tétrazole protégé de formule (IX), en présence d'hydrogénosulfate de tétrabutylammonium,

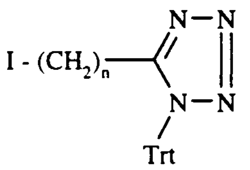

(IX)

dans laquelle Trt représente le groupement triphénylméthyle, pour conduire au composé de formule (X) :

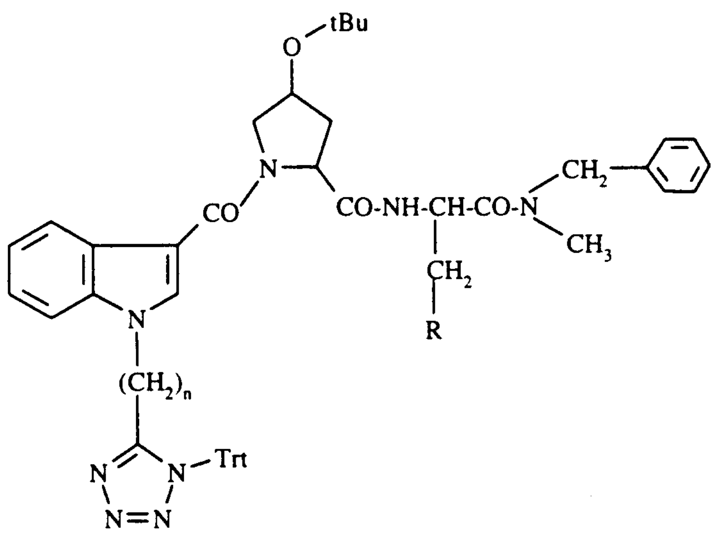

(X)

dans laquelle tBu, R et Trt ont la même signification que précédemment, que l'on déprotège, en milieu acide trifluoroacétique, pour conduire au composé de formule (I),

- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

Le composé de formule (VII) est obtenu par réaction d'un amino-acide protégé de formule (XI) :

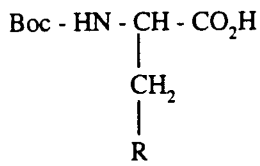

(XI)

dans laquelle R a la même signification que dans la formule (I) et Boc représente un groupement tert-butoxycarbonyle sur la N-méthylbenzylamine, selon une réaction classique de couplage peptidique comme celle décrite par W. KONIG et R. GEIGER (Ber., 103, 788, 1970),
pour conduire au composé de formule (XII) :

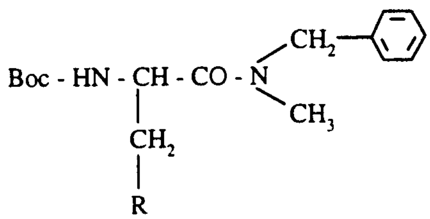

(XII)

dans laquelle Boc et R ont la même signification que précédemment, que l'on déprotège en milieu chlorhydrique, pour conduire au composé de formule (VII).

Le composé de formule (IX) est obtenu par réaction d'un nitrile de formule (XIII) :

$$Cl - (CH_2)_n - CN \qquad (XIII)$$

dans laquelle n a la même signification que dans la formule (I),
avec de l'azoture de sodium en présence de chlorure d'aluminium, sous atmosphère inerte,
pour conduire au composé de formule (XIV) :

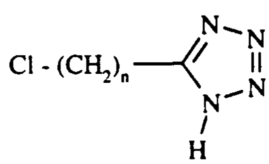

(XIV)

dans laquelle n a la même signification que dans la formule (I),
que l'on fait réagir avec le chlorure de triphénylméthyle en présence de triéthylamine,
pour conduire au composé de formule (XV) :

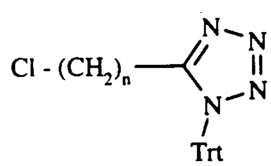

(XV)

dans laquelle n et Trt ont la même signification que précédemment,
qui est ensuite traité avec de l'iodure de sodium, pour conduire au dérivé iodé de formule (IX) correspondant.

Les composés de l'invention possèdent des propriétés pharmacologiques très intéressantes. Ce sont des ligands spécifiques des récepteurs des neurokinines qui possèdent en particulier des propriétés antagonistes particulièrement intenses vis-à-vis des récepteurs $NK_1$. Les récepteurs $NK_1$ seraient plus particulièrement impliqués dans la régulation de la transmission de la douleur, de l'oedème induit par augmentation de la vasoperméabilité, des phénomènes secrétoires au niveau trachéo-bronchique et gastro-intestinal, de la salivation, du contrôle ventilatoire et du tonus vasculaire, et de l'activation des cellules participant aux processus inflammatoires. De plus, contrairement à certains dérivés pseudopeptidiques antagonistes de substance P, ces composés sont dépourvus d'effet dégranulant sur les cellules mastocytaires.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,2 et 100 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

Les préparations A et B ne conduisent pas aux composés de l'invention mais à des intermédiaires de synthèse utiles dans la préparation des composés de formule (I).

Dans les exemples et préparations ci-dessous, les acides aminés dont les abréviations commencent par une lettre majuscule sont de configuration L. Les acides aminés dont les abréviations commencent par une lettre minuscule sont de configuration D.

Les abréviations utilisées dans les exemples et les préparations sont les suivantes:

**Nal-NMeBzl** représente le résidu **(L)-N-méthylbenzylamino-napth-2-ylalanyle** de formule :

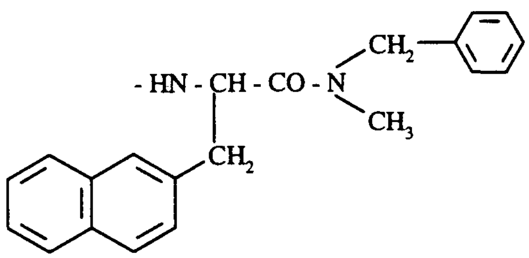

**Tna-NMeBzl** représente le résidu **(L)-N-méthylbenzylamino-5,6,7,8-tétrahydronapht-2-ylalanyle** de formule :

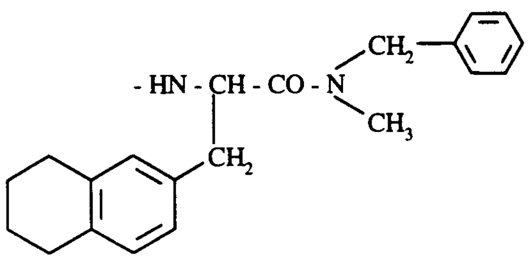

**Thn-NMeBzl** représente le résidu **(L)-N-méthylbenzylamino-1,2,3,4-tétrahydronapht-2-ylalanyle** de formule :

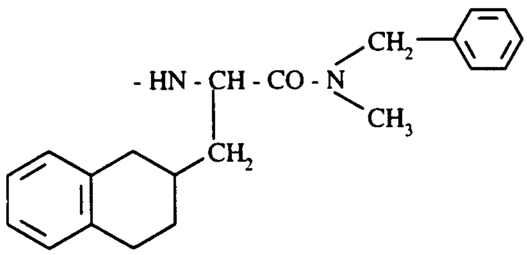

**Thi-NMeBzl** représente le résidu **(L)-N-méthylbenzylaminothién-2-ylalanyle** de formule :

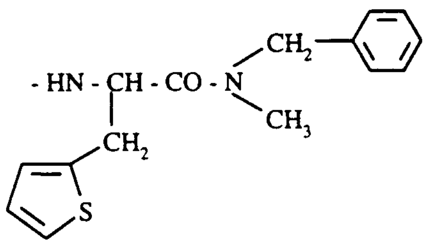

**Phe** représente le résidu **phénylalanyle,**

**Cha-NMeBzl** représente le résidu **(L)-N-méthylbenzylamino-cyclohexylalanyle** de formule :

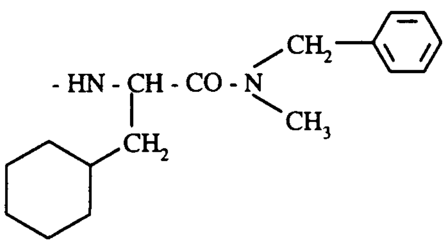

**Boc** représente le groupement **tert-butoxycarbonyle,**

**Hyp** représente le résidu **(R)-4-hydroxy-L-prolyle** de formule :

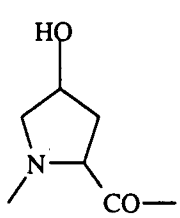

**Fmoc** représente le groupement **9-fluorénylméthoxycarbonyle**

**tBu** représente le groupement **tert-butyle**

**H-Hyp(tBu)-OBzl** représente le résidu de formule :

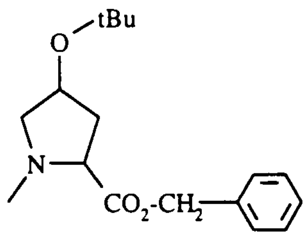

***Préparation A** : H-Nal-NMeBzl, chlorhydrate*

***Stade A** : (L)-Boc-Nal-NMeBzl*

A 30 $cm^3$ de dichlorométhane sont ajoutées successivement 12,6 mmoles de (L)Boc-Nal-OH, 13,9 mmoles de 2-(1H-benzotriazol-1-yl)-1,1,3,3-tétraméthyluronium, tétrafluoroborate puis 12,6 mmoles de N-méthylbenzylamine. Au mélange placé sous agitation dans un bain de glace, 27,7 mmoles de N,N-diisopropyléthylamine sont ajoutées. Après 1 heure d'agitation à 0°C, le mélange est amené à température ambiante et maintenu 20 heures sous agitation. Après concentration, l'huile résiduelle est reprise par de l'acétate d'éthyle. La solution est lavée par du bicarbonate de soude puis par une solution d'acide citrique à 5 % et enfin par une solution saturée de chlorure de sodium. La phase organique séchée et évaporée conduit au produit attendu sous forme d'huile.

***Stade B**: H-Nal-NMeBzl, chlorhydrate*

Le produit attendu est obtenu par traitement du composé obtenu au stade précédent par une solution d'acide chlorhydrique 6N dans de l'acétate d'éthyle, pendant 90 minutes. Après évaporation du solvant, le solide obtenu est repris par de l'éther puis du pentane. Le produit attendu est alors obtenu par filtration du précipité et séchage de celui-ci.

***Préparation B** : 5-(4-Iodobutyl)-1-triphénylméthyl-1H-tétrazole*

***Stade A** : 5-(4-Chlorobutyl)tétrazole*

100 mmoles de 5-chlorovaléronitrile sont placées sous atmosphère inerte dans 100 ml de tétrahydrofurane anhydre dans un bain de glace. 445 mmoles d'azoture de sodium puis 100 mmoles de chlorure d'aluminium sont alors ajoutées par petites fractions. Le mélange réactionnel est porté à 80°C et laissé au reflux, sous agitation, pendant 16 heures. Le complexe formé est alors hydrolysé par addition, goutte à goutte, de 150 $cm^3$ d'acide chlorhydrique à 5%. La phase aqueuse résultante est extraite par de l'acétate d'éthyle et après lavage de la phase organique par de l'eau puis par une solution saturée en chlorure de sodium, séchage et évaporation, le résidu obtenu est repris par du pentane. Le produit attendu est alors obtenu après filtration du précipité formé.

***Stade B** : 5-(4-Chlorobutyl)-1-triphénylméthyl-1H-tétrazole*

A une solution contenant 68 mmoles du composé obtenu au stade précédent dans 80 ml de chloroforme, sont ajoutées successivement 68 mmoles de triéthylamine puis 68 mmoles de chlorure de triphénylméthyle. La solution est laissée 15 heures, sous agitation, à température ambiante. Après évaporation, l'huile résiduelle est reprise par de l'acétate d'éthyle. La solution est lavée au bicarbonate de soude à 5 % puis par de l'acide citrique à 5 % et enfin par une solution saturée de chlorure de sodium. Après séchage et évaporation, le produit attendu, sous forme solide, est obtenu après reprise dans du pentane, filtration et séchage.

*Stade C : 5-(4-Iodobutyl)-1-triphénylméthyl-1H-tétrazole*

A 25 mmoles du composé obtenu au stade précédent dans 100 ml d'acétone anhydre, sont ajoutées 25 mmoles d'iodure de sodium. Le mélange est porté à 60°C et laissé 15 heures au reflux. Après filtration du précipité formé, le filtrat concentré sous vide conduit au produit attendu.

*EXEMPLE 1 : {1-[4-(1H-Tétrazol-5-yl)butyl]indol-3-yl}carbonyl-Hyp-Nal-NMeBzl, sel de potassium*

*Stade A : Fmoc-Hyp(tBu)-OBzl*

A un mélange contenant 61 mmoles de Fmoc-Hyp(tBu)-OH, 65 mmoles d'alcool benzylique et 27 mmoles de 4-diméthylaminopyridine dans 150 ml de dichlorométhane refroidi dans un bain de glace sont ajoutées 65 mmoles de N,N-dicyclohexylcarbodiimide.Après une heure à 0°C, le mélange est amené à température ambiante et laissé 18 heures sous agitation. Le précipité de dicyclohexylurée est filtré et le filtrat est évaporé. L'huile résiduelle est reprise dans de l'acétate d'éthyle. La solution est lavée par du bicarbonate de soude à 5 %, par de l'acide citrique à 5 % puis par une solution saturée en chlorure de sodium. Après séchage et évaporation, on obtient le produit attendu sous forme d'huile.

*Stade B : H-Hyp(tBu)-OBzl*

L'huile obtenue au stade précédent est traitée par 240 ml d'une solution à 20 % de pipéridine dans de l'éther éthylique, pendant 2 heures 30 minutes. Après concentration, l'huile résiduelle est purifiée par chromatographie sur colonne de silice en utilisant comme éluant l'acétate d'éthyle et conduit au produit attendu sous forme d'huile.

*Stade C: (Indol-3-yl)carbonyl-Hyp(tBu)-OBzl*

A un mélange contenant 50 mmoles de produit obtenu au stade précédent, 65 mmoles d'acide indole-3-carboxylique et 75 mmoles de bromo-tris-pyrrolidino-phosphonium hexafluoro phosphate dans 80 ml de dichlorométhane refroidi dans un bain de glace sont ajoutées 100 mmoles de N,N-diisopropyléthylamine. Après 30 minutes à 0°C, le mélange est amené à température ambiante et maintenu 17 heures sous agitation. Après évaporation, l'huile résiduelle est reprise par de l'acétate d'éthyle. La solution est lavée par du bicarbonate de soude à 5 %, par de l'acide citrique à 5 % et par une solution saturée de chlorure de sodium. Le produit attendu cristallise et est isolé par filtration.

*Stade D: (Indol-3-yl)carbonyl-Hyp(tBu)-OH*

23 mmoles du produit obtenu au stade précédent sont dissoutes à chaud dans 1 litre de méthanol anhydre. Après retour à température ambiante, sous atmosphère inerte, on ajoute 50 ml d'acide acétique glacial et 1 g de palladium sur charbon à 1 %. La suspension est hydrogénée 20 heures à température et pression ambiantes. Le catalyseur est alors filtré, rincé au méthanol et le filtrat évaporé. Le résidu est repris par de l'acétate d'éthyle et conduit au produit attendu qui cristallise et est filtré.

*Stade E : (Indol-3-yl)carbonyl-Hyp(tBu)-Nal-NMeBzl*

A un mélange contenant 9,5 mmoles du produit obtenu au stade précédent, 10,5 mmoles du composé décrit dans la préparation A, 10,5 mmoles de 2-(1H-benzotriazol-1-yl)-1,1,3,3-tétraméthyluronium tétrafluoroborate, 10,5 mmoles d'hydroxybenzotriazole dans 50 ml de dichlorométhane refroidi dans un bain de glace, sont ajoutées 21 mmoles de N,N-diisopropyléthylamine. Après une heure à 0°C, le mélange est amené à température ambiante et agité pendant 16 heures. Après concentration, le résidu est repris par de l'acétate d'éthyle. La solution est lavée par du bicarbonate de soude à 5 %, de l'acide citrique à 5 % et par une solution saturée de chlorure de sodium. Le produit attendu cristallise et est filtré.

*Stade F : {1-[4-(4-Triphénylméthyl-1H-tétrazol-5-yl)butyl]indol-3-yl}carbonyl-Hyp(tBu)-Nal-NMeBzl*

A une solution contenant 4,76 mmoles du composé obtenu au stade précédent et 0,05 mmole d'hydrogénosulfate de tétrabutylammonium dans 70 ml de dichlorométhane sont ajoutées 19 mmoles de soude en pastilles finement broyées. La suspension est agitée 5 minutes et 14,3 mmoles du composé obtenu dans la préparation B sont ajoutées. L'ensemble est maintenu sous agitation 24 heures. Après évaporation du solvant, le résidu est repris par de l'acétate d'éthyle. La solution est lavée par du bicarbonate de soude à 5 %, de l'acide citrique à 5 % puis par une solution saturée de chlorure de sodium. Après séchage et évaporation, le résidu est purifié par chromatographie sur

colonne de silice en utilisant comme éluant de l'acétate d'éthyle et conduit au produit attendu sous forme de poudre.

***Stade G**: {1-[4-(1H-Tétrazol-5-yl)butyl]indol-3-yl}carbonyl-Hyp-Nal-NMeBzl*

3,01 mmoles du composé obtenu au stade précédent sont traitées par 500 ml d'un mélange acide trifluoroacétique/dichlorométhane (50/50), à température ambiante, pendant 3 heures. Après concentration, l'huile résiduelle est reprise par un mélange éther éthylique/pentane et conduit à un précipité qui est filtré et purifié par HPLC préparative sur phase inverse $C_{18}$ en utilisant comme solvant un système eau/acétonitrile dont le pourcentage d'acétonitrile varie de 30 à 40 % en 1 heure. Cette purification conduit au produit attendu qui est lyophilisé.

***Stade H : {1-[4-(1H-Tétrazol-5-yl)butyl]indol-3-yl}carbonyl-Hyp-Nal-NMeBzl, sel de potassium***

Le sel de potassium du produit décrit au stade précédent est obtenu par addition de 2,08 mmoles d'une solution d'hydroxyde de potassium 0,1 N à une solution refroidie contenant 1,89 mmoles du composé obtenu au stade précédent dans un mélange eau/acétonitrile. Ce sel est alors lyophilisé.

| *Microanalyse élémentaire :* | | | |
|---|---|---|---|
| | *C %* | *H %* | *N %* |
| *calculé* | *60,20* | *5,89* | *13,96* |
| *trouvé* | *59,82* | *5,58* | *13,61* |

***Exemple 2 : {1-[4-(1H-tétrazol-5-yl)butyl]indol-3-yl}carbonyl-Hyp-Tna-NMeBzl, sel de potassium***

et

***Exemple 3 : {1-[4-(1H-tétrazol-5-yl)butyl]indol-3-yl}carbonyl-Hyp-Thn-NMeBzl, sel de potassium***

***Stade A : (L)-Boc-Tna-NMeBzl et [(L,L)+(L,D)]-Boc-Thn-NMeBzl***

A une solution de 7,17 mmoles de (L)-Boc-Nal-NMeBzl dans 20 $cm^3$ de méthanol sont ajoutés successivement 10 gouttes d'acide acétique glacial puis 1,5 g de palladium sur charbon. Le mélange est hydrogéné pendant 3 jours à température et pression ambiantes. Après élimination du catalyseur par filtration et évaporation du solvant, les produits attendus sont obtenus sous forme d'huile incolore, dans des proportions : (L)-Boc-Tna-NMeBzl/(L,L)-Boc-Thn-NMeBzl/(L,D)-Boc-Thn-NMeBzl : 60/20/20.

***Stade B : H-Tna-NMeBzl, trifluoroacétate + H-Thn-NMeBzl, trifluoroacétate***

L'huile précédente est traitée par 100 $cm^3$ d'un mélange acide trifluoroacétique/ dichlorométhane (50/50) et laissée sous agitation à température ambiante pendant 2 heures. Après concentration sous vide et trituration dans le pentane, le mélange déprotégé est obtenu sous forme d'huile.

***Stade C : (Indol-3-yl)carbonyl-Hyp(tBu)-Tna-NMeBzl + (Indol-3-yl)carbonyl-Hyp(tBu)-Thn-NMeBzl***

A un mélange contenant 2 mmoles de (indol-3-yl)carbonyl-Hyp(tBu)-OH, 2 mmoles du composé décrit dans le stade B précédent, 2,2 mmoles de TBTU, 2,2 mmoles de HOBt dans 10 ml de dichlorométhane, sont ajoutées 4,4 mmoles de N,N-diisopropyléthylamine. La solution résultante est laissée sous agitation pendant 8 heures puis est reprise dans de l'acétate d'éthyle. Après lavage de la phase organique par de l'eau, du bicarbonate de soude à 5 %, de l'acide citrique à 5 % et par une solution saturée de chlorure de sodium, séchage et concentration sous vide, la mousse cristalline obtenue est purifiée sur silice avec l'éluant acétate d'éthyle 100 %. Une purification supplémentaire par HPLC préparative sur phase inverse C18, en utilisant les éluants A : eau/acide trifluoroacétique 0,1 % ; B : acétonitrile/acide trifluoroacétique 0,1 % selon un gradient 35 % de B à 65 % de B en 90 min, permet de séparer les deux dérivés contenant soit Tna, soit Thn. Dans ces conditions par contre, il n'a pas été possible de séparer les deux diastéréoisomères de l'adduit Thn.

***Exemple 2* : {1-[4-(1H-tétrazol-5-yl)butyl]indol-3-yl}carbonyl-Hyp-Tna-NMeBzl, sel de potassium**

Le produit attendu est préparé selon les protocoles F, G et H de l'exemple 1 à partir de (indol-3-yl)carbonyl-Hyp(tBu)-Tna-NMeBzl obtenu au stade C précédent.

| *Microanalyse élémentaire :* | | | |
|---|---|---|---|
| | *C %* | *H %* | *N %* |
| *calculé* | *64,84* | *6,12* | *15,12* |
| *trouvé* | *65,32* | *5,88* | *14,46* |

*Spectre de masse FAB : $[M+H]^+$ m/z = 741*

***Exemple 3* : {1-[4-(1H-tétrazol-5-yl)butyl]indol-3-yl)carbonyl-Hyp-Thn-NMeBzl, sel de potassium**

Le produit attendu est préparé selon les protocoles F, G et H de l'exemple 1 à partir de (indol-3-yl)carbonyl-Hyp(tBu)-Thn-NMeBzl obtenu au stade C précédent.

*Spectre de masse : FAB : $[M+H]^+$ m/z = 741*

***Exemple 4* : {1-[(1H-tétrazol-5-yl)méthyl]indol-3-yl}carbonyl-Hyp-Nal-NMeBzl, sel de potassium**

Ce composé a été préparé selon le même protocole que dans l'exemple 1, en utilisant le 5-iodométhyl-1-triphénylméthyl-1H-tétrazole obtenu selon la préparation B à partir du chloroacétonitrile.

| *Microanalyse élémentaire :* | | | |
|---|---|---|---|
| | *C %* | *H %* | *N %* |
| *calculé* | *63,96* | *5,08* | *16,13* |
| *trouvé* | *63,57* | *5,01* | *15,93* |

*Spectre de masse FAB : $[M+H]^+$ m/z = 695*

***Exemple 5* : {1-[2-(1H-tétrazol-5-yl)éthyl]indol-3-yl}carbonyl-Hyp-Nal-NMeBzl, sel de potassium**

***Stade A* : [1-(2-cyanoéthyl)indol-3-yl]carbonyl-Hyp(tBu)-Nal-NMeBzl**

A une solution contenant 0,79 mmoles d'(indol-3-yl)carbonyl-Hyp(tBu)-Nal-NMeBzl (stade E de l'exemple 1) et 0,87 mmoles de 3-bromopropionitrile dans 10 ml de tétrahydrofurane anhydre sont ajoutées par petites portions 1,58 mmoles d'hydrure de sodium. Après 24 heures sous agitation à température ambiante, le solvant est éliminé sous vide et le résidu repris dans de l'acétate d'éthyle. La solution résultante est lavée par de l'eau, du bicarbonate de soude 5 %, de l'acide citrique 5 % et par une solution saturée en chlorure de sodium puis séchée sur $Na_2SO_4$. Après évaporation du solvant, le mélange est purifié par flash-chromatographie sur silice avec l'éluant dichlorométhane/acétone 80/20. Les fractions sont concentrées sous vide pour conduire au produit attendu sous forme de poudre blanche.

***Stade B* : {1-[2-(1H-tétrazol-5-yl)éthyl]indol-3-yl}carbonyl-Hyp(tBu)-Nal-NMeBzl**

A une solution contenant 0,44 mmoles du peptide obtenu lors du stade précédent dans 10 ml de toluène sont

ajoutées 1,75 mmoles de triméthylsilylazide et 0,09 mmoles d'oxyde de dibutylétain. Le mélange réactionnel est maintenu sous agitation à 80°C pendant 24 heures puis le toluène est évaporé. Le résidu est repris par deux fois dans le méthanol puis est concentré sous vide et repris dans l'acétate d'éthyle. La solution est lavée par de l'acide citrique 5 % et par une solution saturée en chlorure de sodium, séchée sur $Na_2SO_4$ et concentrée sous vide pour conduire au produit attendu sous forme de poudre blanche.

***Stade C*: {1-[2-(1H-tétrazol-5-yl)éthyl]indol-3-yl}carbonyl-Hyp-Nal-NMeBzl**

0,35 mmoles du composé obtenu au stade précédent sont traitées par 20 ml d'un mélange acide trifluoroacétique/dichlorométhane (50/50) à température ambiante pendant 5 heures. Après concentration, l'huile résultante est reprise par de l'éther éthylique et conduit à un précipité qui est filtré et purifié par HPLC préparative sur phase inverse $C_{18}$ en utilisant comme solvant un système eau/acétonitrile dont le pourcentage d'acétonitrile varie de 35 à 50 % en 30 min. Cette purification conduit au produit qui est lyophilisé.

***Stade D* : {1-[2-(1H-tétrazol-5-yl)éthyl]indol-3-yl}carbonyl-Hyp-Nal-NMeBzl, sel de potassium**

Le sel de potassium du produit décrit au stade précédent est obtenu par addition de 0,111 mmoles d'une solution d'hydroxyde de potassium 0,1 N à une solution refroidie de 0,110 mmoles du composé obtenu au stade précédent dans un mélange eau/acétonitrile. Ce sel est alors lyophilisé.

| *Microanalyse élémentaire :* | | | |
|---|---|---|---|
| | *C %* | *H %* | *N %* |
| *calculé* | *64,39* | *5,26* | *15,81* |
| *trouvé* | *63,75* | *5,00* | *15,26* |

*Spectre de masse FAB : $[M+H]^+$ m/z = 709*

***Exemple 6*: {1-[3-(1H-tétrazol-5-yl)propyl]indol-3-yl}carbonyl-Hyp-Nal-NMeBzl, sel de potassium**

Ce composé a été préparé selon le même protocole que dans l'exemple 1, en utilisant le 5-(3-iodopropyl)-1-triphénylméthyl-1H-tétrazole obtenu comme décrit dans la préparation B à partir du 4-chlorobutyronitrile.

| *Microanalyse élémentaire :* | | | |
|---|---|---|---|
| | *C %* | *H %* | *N %* |
| *calculé* | *64,80* | *5,44* | *15,50* |
| *trouvé* | *64,78* | *5,24* | *15,27* |

*Spectre de masse FAB : $[M+H]^+$ m/z = 723*

***Exemple 7* : {1-[5-(1H-tétrazol-5-yl)pentyl]indol-3-yl}carbonyl-Hyp-Nal-NMeBzl, sel de potassium**

Ce produit a été préparé selon le même protocole que pour le composé de l'exemple 5, en partant du 6-bromocapronitrile.

| *Microanalyse élémentaire :* | | | |
|---|---|---|---|
| | *C %* | *H %* | *N %* |
| *calculé* | *65,58* | *5,77* | *14,92* |
| *trouvé* | *64,85* | *5,61* | *14,38* |

*Spectre de masse FAB : $[M+H]^+$ m/z = 751*

Les exemples 8 à 11 ont été préparés selon le procédé décrit dans l'exemple 1 à partir des produits de départ correspondants obtenus selon le procédé décrit dans la préparation A.

***Exemple 8* : {1-[4-(1H-Tétrazol-5-yl)butyl]indol-3-yl}carbonyl-Hyp-Thi-NMeBzl, sel de potassium**

| *Microanalyse élémentaire :* | | | |
|---|---|---|---|
| | *C %* | *H %* | *N %* |
| *calculé* | *59,02* | *5,24* | *16,20* |
| *trouvé* | *59,02* | *5,12* | *15,68* |

*Spectre de masse FAB : $[M+H]^+$ m/z = 655*

***Exemple 9* : {1-[4-(1H-Tétrazol-5-yl)butyl]indol-3-yl}carbonyl-Hyp-(4-chloro)Phe-NMeBzl, sel de potassium**

*Spectre de masse FAB : $[M+H]^+$ m/z = 721*

***Exemple 10* : {1-[4-(1H-Tétrazol-5-yl)butyl]indol-3-yl}carbonyl-Hyp-(3,4-diméthoxy)Phe-NMeBzl, sel de potassium**

*Spectre de masse FAB : $[M+H]^+$ m/z = 747*

***Exemple 11* : {1-[4-(1H-Tétrazol-5-yl)butyl]indol-3-yl}carbonyl-Hyp-Cha-NMeBzl, sel de potassium**

*Spectre de masse FAB : $[M+H]^+$ m/z = 693*

**Etude pharmacologique des dérivés de l'invention**

***Exemple 12* : Affinité aux récepteurs humains $NK_1$ et $NK_2$**

L'affinité pour les récepteurs humains $NK_1$ et $NK_2$ a été étudiée sur les lymphoblastes humains IM-9 exprimant

spécifiquement le récepteur $NK_1$ comme décrit par D.G. PAYAN et coll. (J. Biol. Chem. 1986, 261, 14321-14329) et sur les cellules CHO-K1 transfectées avec le récepteur $NK_2$ selon le kit "CellPhect transfection" (Pharmacia).
Les composés de l'invention ont montré une excellente affinité spécifique pour les récepteurs $NK_1$. Les résultats sont regroupés dans le tableau ci-dessous :

| Exemple | NK1 (IM9) KI nM | NK2 (CHO) KI nM |
|---|---|---|
| 1 | 0.04 | 2 400 |
| 2 | 14 | 3 600 |
| 3 | 14 | 1 400 |
| 4 | 0.78 | > 1 000 |
| 5 | 0.63 | > 10 000 |
| 6 | 1.2 | > 1 000 |
| 7 | 5.9 | 10 000 |
| 8 | 110 | > 1 000 |
| 9 | 22 | 3 900 |
| 10 | 180 | > 10 000 |
| 11 | 52 | > 1 000 |

***Exemple 13 : Essais sur le muscle lisse isolé***

Afin d'évaluer l'activité fonctionnelle des composés de l'invention comme antagonistes des neurokinines, trois préparations isolées de muscle lisse ont été utilisées : la veine cave de lapin (VCL), l'artère pulmonaire de lapin sans endothélium (APL) et la veine porte de rat (VPR) dont les réponses contractiles sont respectivement médiées par les récepteurs $NK_1$, $NK_2$ et $NK_3$ comme indiqué par D. JUKIC et coll. (Life Sci. 1991, 49, 1463-1469).
Le pouvoir antagoniste des composés de l'invention a été exprimé sous forme de $pA_2$ tel que défini par O. ARUNLAKSHANA et H.O. SCHILD (Brit. J. Pharmacol. 1959, 14, 48-58).

Les composés de l'invention ont montré une puissante activité antagoniste vis-à-vis des récepteurs $NK_1$ avec une faible activité pour les récepteurs $NK_2$ et $NK_3$. Les résultats obtenus pour les exemples 1, 2 et 3 sont regroupés dans le tableau ci-dessous :

| Exemple | NK1 (VCL) pA2 | NK2 (APL) pA2 | NK3 (VPR) pA2 |
|---|---|---|---|
| 1 | 9.57 | 5.57 | 4.96 |
| 2 | 8.17 | 6.47 | 5.87 |
| 3 | 7.87 | 5.87 | 5.87 |
| 4 | 8.14 | - | 5.84 |
| 6 | 8.46 | 5.20 | - |
| 7 | 7.88 | - | - |

***Exemple 14 : Etude du potentiel antinociceptif - Test d'Eddy chez la souris***

En raison de l'implication de la substance P dans la transmission nociceptive au niveau spinal (M. OTSUKA et S. KONISHI, TINS, 6, 317-320, 1983) et plus particulièrement dans la douleur d'origine thermique (A.W. DUGGAN et coll., Brain Research, 1987, 403, 345-349), l'activité pharmacologique in vivo des composés de l'invention a été recherchée chez la souris sur le test d'hyperalgésie thermique initialement décrit par N.B. EDDY et coll. (J. Pharmacol.

Exp. Ther., 1953, 107, 385-393). Ce test consiste à mesurer le temps de réaction à la chaleur déterminé par le léchage des pattes antérieures chez la souris (CD1 mâle, Ch. River, 25-30 g) placée sur une plaque métallique chauffée à 55°C. Les animaux ont été traités avec les composés de l'invention par voie intraveineuse, 10 minutes avant le passage sur la plaque chauffante.
La moyenne des temps de réaction obtenue pour chaque lot traité (12 souris par lot) a été comparée à la moyenne du lot témoin correspondant. Les résultats sont exprimés sous forme d'$ED_{50}$ qui correspond à la dose augmentant de 50 % le temps de réaction.
Alors que la substance P administrée par voie intra-spinale, diminue le temps de réaction de l'animal, les composés de l'invention ont augmenté ce temps de réaction après injection intraveineuse. Par exemple, le composé de l'exemple 1 a présenté une $ED_{50}$ de 0,018 mg/kg (intervalle de confiance pour P = 0,05 : 0,001- 0,322), l'$ED_{50}$ de la morphine étant de 0,30 mg/kg (0,03- 2,90).
A l'inverse de celle de la morphine, l'activité des composés n'a pas été antagonisée par la naloxone.

***Exemple 15** : Etude de l'inhibition de l'extravasation plasmatique induite par la substance P chez le cobaye*

L'effet des composés sur l'extravasation plasmatique provoquée par injection intraveineuse de substance P (1 µg/kg) chez le cobaye a été évalué au niveau de la vessie, selon la méthode décrite chez le rat par C. GARRET et coll. (Proc. Natl. Acad. Sci., USA 1991, 88, 10208-20212). L'accumulation vésicale de bleu Evan's injecté IV en même temps que la substance P et 10 minutes avant le sacrifice, a été quantifiée par spectrophotométrie après extraction du colorant par l'acétone. L'activité inhibitrice des composés administrés par voie intraveineuse 5 minutes avant la substance P a été exprimée en % d'inhibition par comparaison à un lot contrôle (8 animaux par lot). A titre d'exemple, le composé de l'exemple 1 a exercé une activité de 56 % à la dose de 0,1 mg/kg.

***Exemple 16** : Etude de l'inhibition de la bronchoconstriction induite par la substance P chez le cobaye*

L'étude est réalisée sur des cobayes Hartley mâles (Charles River) de poids moyen 300 à 400g. L'étude est réalisée sur des animaux anesthésiés (carbamate d'éthyle 1,5 g/kg) et curarisés flaxedil (0,2 mg/kg iv) ventilés avec une fréquence de 60 par minute et un volume courant de 10 ml/kg. Les animaux sont pré-traités par pyrilamine (1 mg/kg iv), propanolol (1 mg/kg iv).
Le critère de jugement de la bronchoconstriction est l'augmentation de la pression d'insuflation trachéale (PIT) induite par l'injection de substance P par voie iv à la dose de 2 nM/kg, iv, chaque animal étant son propre témoin. L'injection du produit testé s'effectuant par rapport au temps T0 injection du produit.
Les résultats sont exprimés en pourcentage d'inhibition de la bronchoconstriction induite par la substance P, ce pourcentage étant calculé selon la formule suivante :

$$(\Delta \text{ PIT avant produit} - \Delta \text{ PIT après produit}) / \Delta \text{ PIT avant produit (exprimé en pourcentage).}$$

Les résultats sont regroupés dans le tableau ci-dessous :

| Exemple | % Inhibition dose 1 µmol/kg i.v. |
|---|---|
| 1 | 95 % |
| 4 | 61 % |
| 6 | 60 % |
| 7 | 58 % |

***Exemple 17** : Etude de la dégranulation mastocytaire in vitro*

Les études sont effectuées sur mastocytes péritonéaux de rats mâles : Sprague-Dawley de poids moyen 350 à 400 g et sont euthanasiés par inhalation de $CO_2$. Un lavage péritonéal est effectué avec 20 ml de tampon (NaCl 150 mM, KCl 2.7 mM, $CaCl_2$, 0.9 mM, $Na_2HPO_4$, 3 mM, $KH_2PO_4$, 3.5 mM, Glucose 5.6 mM, albumine de sérum bovin 1 mg/ml, pH 6.8).
Le liquide de lavage est centrifugé à 400 g pendant 10 mn à 4°C et repris dans un tampon à une densité de

$2.10^4$ mastocytes/ml. Les composés sont mis à incuber à concentration croissante pendant 20 mn. La réaction est stoppée en plaçant les tubes sur glace et une centrifugation à 700 g pendant 10 mn à 4°C est effectuée. Le surnageant et le culot sont testés par fluorimétrie après condensation avec O-Phtalaldehyde. L'histamine libérée à partir des cellules dans le surnageant ainsi dosée est exprimée en pourcentage du contenu total d'histamine des cellules. Lors de ce test, le composé de l'exemple 1 n'entraîne pas de dégranulation mastocytaire jusqu'à la concentration de $10^{-4}$M.

**Composition pharmaceutique**

***EXEMPLE 18* : Comprimé : formule de préparation pour 1000 comprimés dosés à 2 mg**

| | |
|---|---|
| Composé de l'exemple 1 | 2 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) :

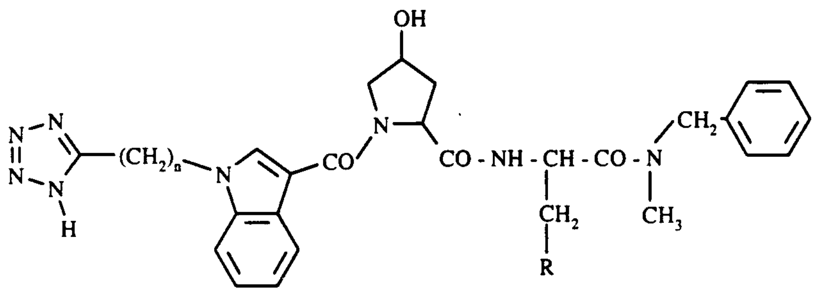

(I)

dans laquelle :

R représente un groupement naphtyle, 5,6,7,8-tétrahydronaphtyle, 1,2,3,4-tétrahydronaphtyle, thiényle, phényle (éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle) ou cycloalkyle ($C_3$-$C_7$),

n représente un entier tel que $1 \leq n \leq 8$,

leurs énantiomères, diastéréoisomères et épimères ainsi que leurs sels d'addition à un acide ou à une base pharmaceutiquement acceptable.

2. Composé de formule (I) selon la revendication 1 tel que n est égal à 4.

3. Composé de formule (I) selon la revendication 1 tel que R représente un groupement naphtyle.

4. Composé de formule (I) selon la revendication 3 tel que R représente un groupement 2-naphtyle.

5. Composé de formule (I) selon la revendication 1 qui est le {1-[4-(1H-tétrazol-5-yl)butyl]indol-3-yl}carbonyl-Hyp-Nal-NMeBzl, ainsi que son sel de potassium correspondant.

6. Procédé de préparation des composés du formule (I) selon la revendication 1 caractérisé en ce que l'on fait réagir une hydroxyproline protégée, de formule (II), dont on a éventuellement séparé les isomères :

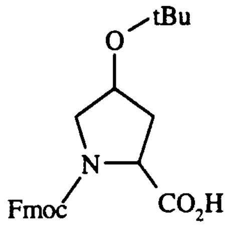

(II)

dans laquelle :

Fmoc représente le groupement 9-fluorénylméthoxycarbonyle,
tBu représente le groupement tert-butyle,
avec de l'alcool benzylique, en présence de 4-diméthylaminopyridine et de N,N-dicyclohexylcarbodiimide, pour conduire au composé de formule (III) :

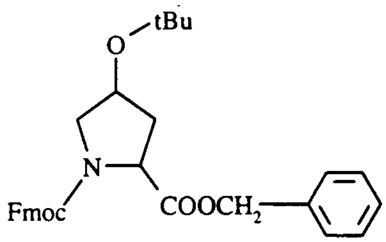

(III)

dans laquelle Fmoc et tBu ont les mêmes significations que précédemment,
dont on déprotège la fonction amine en milieu pipéridine,
pour conduire au composé de formule (IV) :

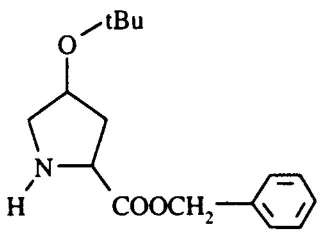

(IV)

dans laquelle tBu a la même signification que précédemment,
que l'on fait réagir avec de l'acide indole-3-carboxylique,
pour conduire au composé de formule (V) :

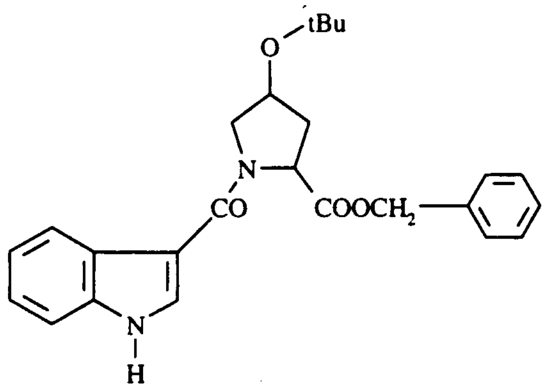

(V)

dans laquelle tBu a la même signification que précédemment,
dont on déprotège la fonction acide carboxylique, par hydrogénation catalytique,
pour conduire au composé de formule (VI) :

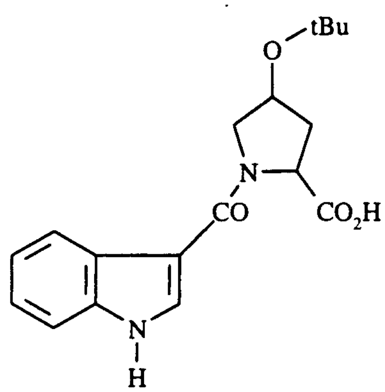

(VI)

dans laquelle tBu a la même signification que précédemment,
que l'on met en réaction avec un amino-acide protégé de formule (VII), dont on a éventuellement séparé les isomères :

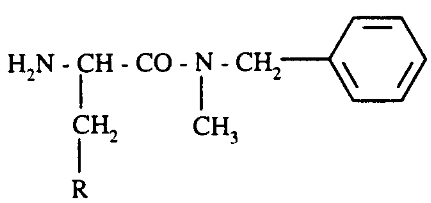

(VII)

dans laquelle R a la même signification que dans la formule (I),
pour conduire au composé de formule (VIII) :

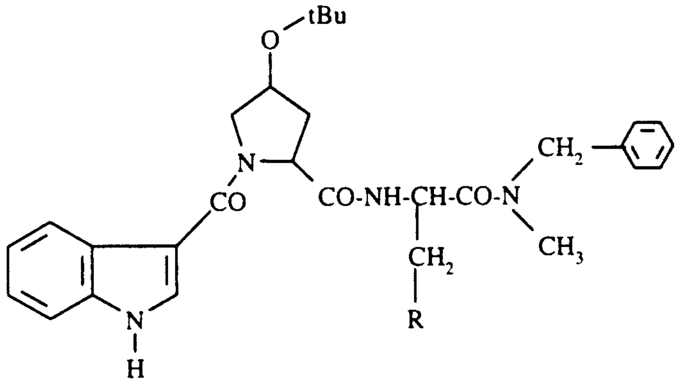

(VIII)

dans laquelle tBu et R ont la même signification que précédemment, que l'on fait réagir sur un tétrazole protégé de formule (IX), en présence d'hydrogénosulfate de tétrabutylammonium,

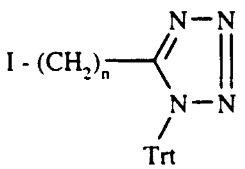

(IX)

dans laquelle Trt représente le groupement triphénylméthyle, pour conduire au composé de formule (X) :

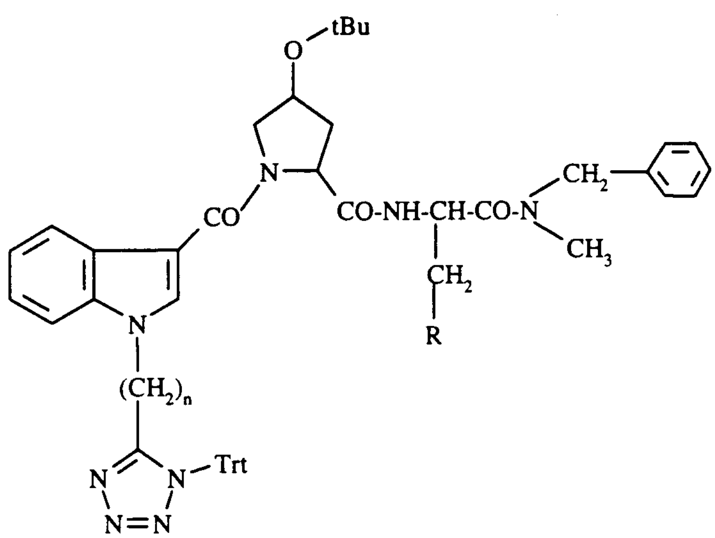

(X)

dans laquelle tBu, R et Trt ont la même signification que précédemment, que l'on déprotège, en milieu acide trifluoroacétique, pour conduire au composé de formule (I),

- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

7. Compositions pharmaceutiques contenant comme principe actif au moins un composé selon l'une quelconque des revendications 1 à 5, seul ou en combinaison avec un ou plusieurs excipients ou véhicules inertes, non toxiques, pharmaceutiquement acceptables.

8. Compositions pharmaceutiques selon la revendication 7 contenant au moins un principe actif selon l'une quelconque des revendications 1 à 5 utiles comme antagonistes de substance P dans le traitement de la douleur, de l'inflammation, des troubles gastro-intestinaux ou urinaires, de l'asthme, des allergies de la migraine et des maladies du système nerveux central.

## Claims

1. Compound of formula (I):

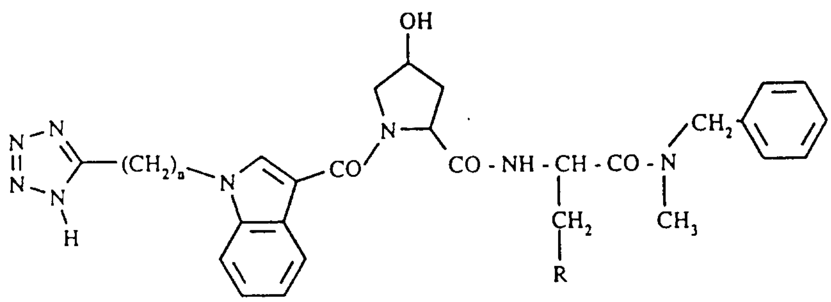

(I)

in which:

R represents a group: naphthyl, 5,6,7,8-tetrahydronaphthyl, 1,2,3,4-tetrahydronaphthyl, thienyl, phenyl (optionally substituted by one or more halogen atoms or linear or branched ($C_1$-$C_6$)alkyl, linear or branched ($C_1$-$C_6$)alkoxy hydroxy, trihalomethyl groups) or ($C_3$-$C_7$)cycloalkyl,

n represents an integer such that $1 \leq n \leq 8$,

their enantiomers, diastereoisomers and epimers, and addition salts thereof with a pharmaceutically acceptable acid or base.

2. Compound of formula (I) according to claim 1 in which n is 4.

3. Compound of formula (I) according to claim 1 in which R represents a naphthyl group.

4. Compound of formula (I) according to claim 3 in which R represents a 2-naphthyl group.

5. Compound of formula (I) according to claim 1 which is {1-[4-(1H-tetrazol-5-yl)-butyl]indol-3-yl}carbonyl-Hyp-Nal-NMeBzl and its corresponding potassium salt.

6. Process for the preparation of compounds of formula (I) according to claim 1, characterised in that a protected hydroxyproline of formula (II), which has optionally been separated into its isomers:

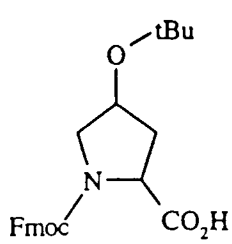

(II),

in which:

Fmoc represents a 9-fluorenylmethoxycarbonyl group, and
tBu represents a tert-butyl group,

is reacted with benzyl alcohol, in the presence of 4-dimethylaminopyridine and N,N-dicyclohexylcarbodiimide, to yield the compound of formula (III):

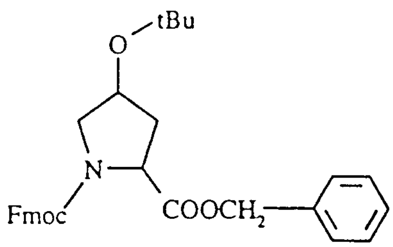

(III)

in which Fmoc and tBu are as defined above,
the amine function of which is deprotected in a piperidine medium to yield the compound of formula (IV):

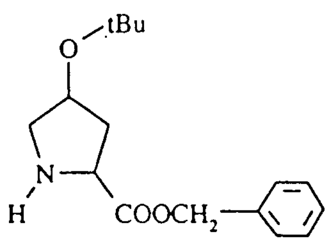

(IV)

in which tBu is as defined above,
which is reacted with indole-3-carboxylic acid to yield the compound of formula (V):

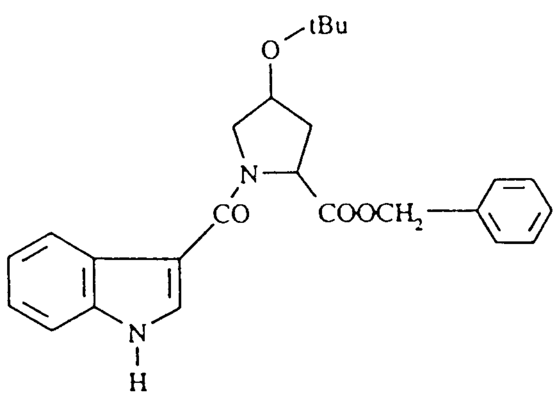

(V)

in which tBu is as defined above,
the carboxylic acid function of which is deprotected, by catalytic hydrogenation, to yield the compound of formula (VI):

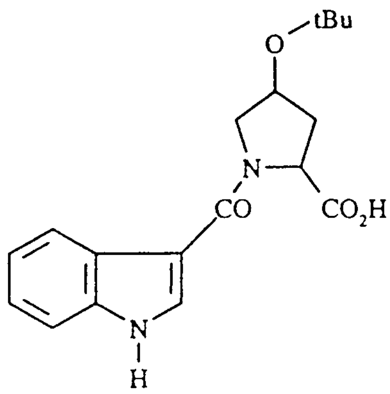

(VI)

in which tBu is as defined above,
which is reacted with a protected amino acid of formula (VII), which has optionally been separated into its isomers:

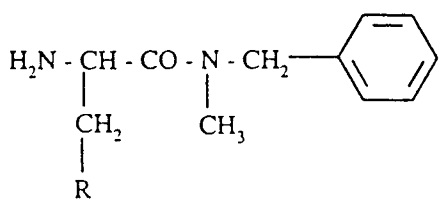

(VII),

in which R is as defined in formula (I),
to yield a compound of formula (VIII):

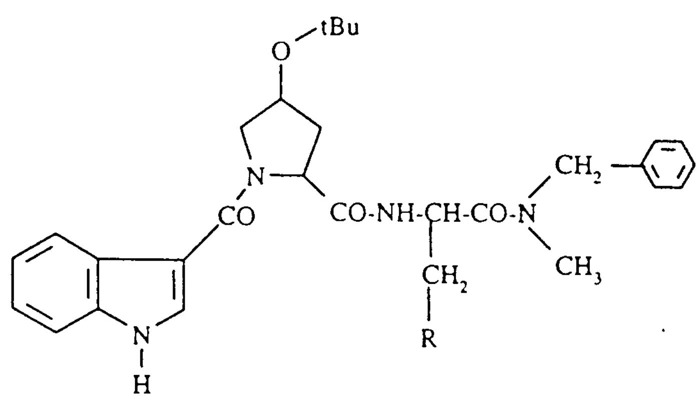

(VIII)

in which tBu and R are as defined above,
which is reacted with a protected tetrazole of formula (IX), in the presence of tetrabutylammonium hydrogen sulphate,

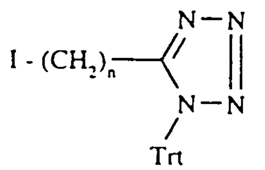

(IX),

in which Trt represents a triphenylmethyl group,
to yield a compound of formula (X):

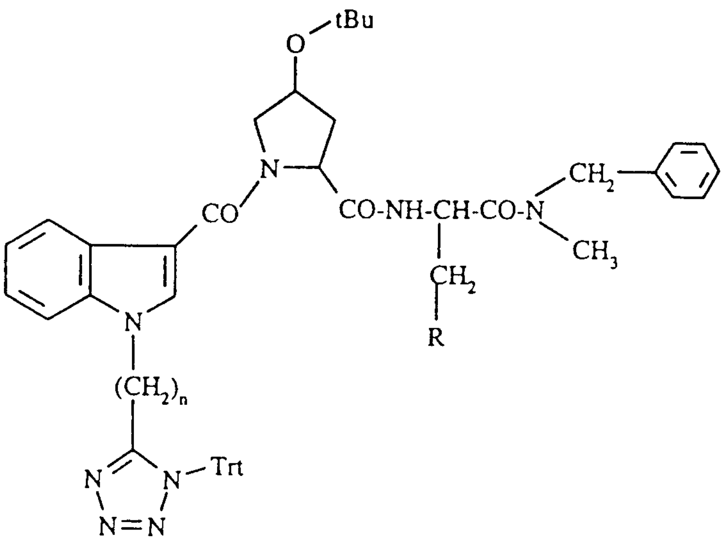

(X)

in which tBu, R and Trt are as defined above,
which is deprotected, in a trifluoroacetic acid medium, to yield a compound of formula (I),

- which may, where appropriate, be purified by a conventional purification technique,
- which is, where appropriate, separated into its isomers by a conventional separation technique,
- which is, if desired, converted into its addition salts with a pharmaceutically acceptable base.

**7.** Pharmaceutical compositions comprising as active ingredient at least one compound according to any one of claims 1 to 5, on its own or in combination with one or more inert, non-toxic, pharmaceutically acceptable excipients or carriers.

**8.** Pharmaceutical compositions according to claim 7 comprising at least one active ingredient according to any one of claims 1 to 5 for use as substance P antagonists in the treatment of pain, inflammation, gastrointestinal or urinary disorders, asthma, allergies, migraine, and diseases of the central nervous system.

**Patentansprüche**

**1.** Verbindung der Formel (I):

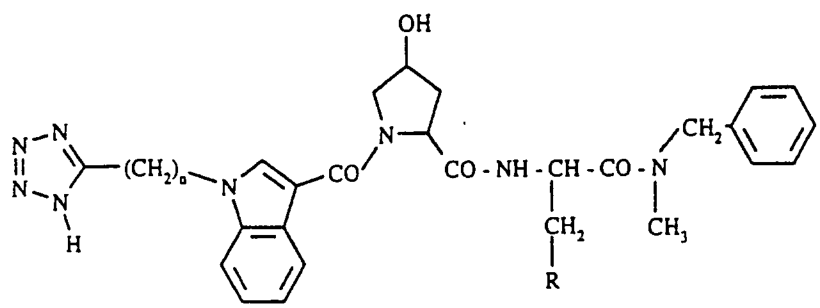

(I)

in der:

R eine Naphthylgruppe, 5,6,7,8-Tetrahydronaphthylgruppe, 1,2,3,4-Tetrahydronaphthylgruppe, Thienylgruppe, Phenylgruppe (die gegebenenfalls durch ein oder mehrere Halogenatome oder geradkettige oder verzweigte ($C_1$-$C_6$)-Alkylgruppen, geradkettige oder verzweigte ($C_1$-$C_6$)-Alkoxygruppen, Hydroxygruppen oder Trihalogenmethylgruppen substituiert ist) oder ($C_3$-$C_7$)-Cycloalkylgruppe und

n eine ganze Zahl mit einem Wert von $1 \leq n \leq 8$ bedeuten, deren Enantiomere, Diastereoisomere und Epimere sowie deren Additionssalze mit einer pharmazeutisch annehmbaren Säure oder Base.

**2.** Verbindung der Formel (I) nach Anspruch 1, worin n den Wert 4 besitzt.

**3.** Verbindung der Formel (I) nach Anspruch 1, worin R eine Naphthylgruppe darstellt.

**4.** Verbindung der Formel (I) nach Anspruch 3, worin R eine 2-Naphthylgruppe darstellt.

**5.** Verbindung der Formel (I) nach Anspruch 1, nämlich {1-[4-(1H-Tetrazol-5-yl)-butyl]-indol-3-yl}-carbonyl-Hyp-Nal-NMeBzl sowie dessen Kaliumsalz.

**6.** Verfahren zur Herstellung der Verbindungen der Formel (I) nach Anspruch 1, dadurch gekennzeichnet, daß man ein geschütztes Hydroxyprolin der Formel (II), das man gegebenenfalls in die Isomeren aufgetrennt hat:

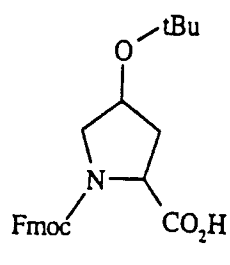

(II)

in der:

Fmoc die 9-Fluorenylmethoxycarbonylgruppe und
tBu die tert.-Butylgruppe bedeuten,
mit Benzylalkohol in Gegenwart von 4-Dimethylaminopyridin und N,N-Dicyclohexylcarbodiimid umsetzt
zur Bildung der Verbindung der Formel (III):

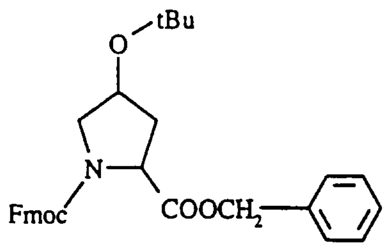

(III)

in der Fmoc und tBu die oben angegebenen Bedeutungen besitzen, von der man die Schutzgruppe der Aminogruppe in Piperidinmedium abspaltet,
zur Bildung der Verbindung der Formel (IV):

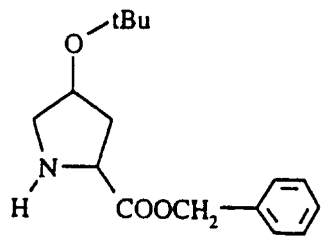

(IV)

in der tBu die oben angegebenen Bedeutungen besitzt,
welche man mit Indol-3-carbonsäure umsetzt
zur Bildung der Verbindung der Formel (V):

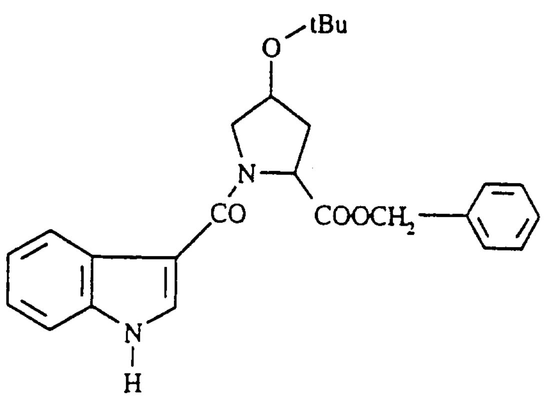

(V)

in der tBu die oben angegebenen Bedeutungen besitzt,
wovon man die Schutzgruppe der Carbonsäurefunktion durch katalytische Hydrierung abspaltet,
zur Bildung der Verbindung der Formel (VI):

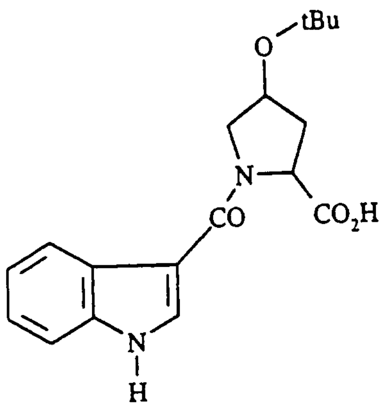

(VI)

in der tBu die oben angegebenen Bedeutungen besitzt,
welche man mit einer geschützten Aminosäure der Formel (VII), die man gegebenenfalls in die Isomeren aufgetrennt hat:

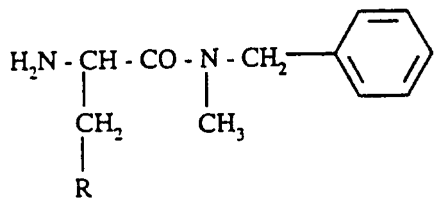

(VII)

in der R die bezüglich der Formel (I) angegebenen Bedeutungen besitzt, umsetzt zur Bildung der Verbindung der Formel (VIII):

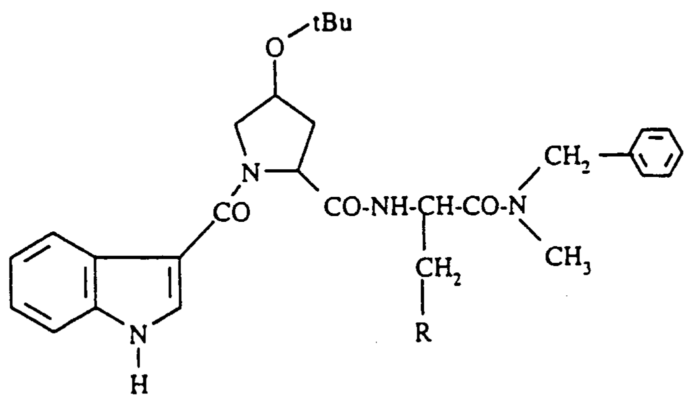

(VIII)

in der tBu und R die oben angegebenen Bedeutungen besitzen,
welche man in Gegenwart von Tetrabutylammoniumhydrogensulfat mit einem geschützten Tetrazol der Formel (IX)

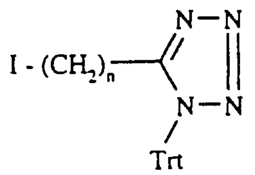

(IX)

in der Trt eine Triphenylmethylgruppe darstellt, umsetzt zur Bildung der Verbindung der Formel (X):

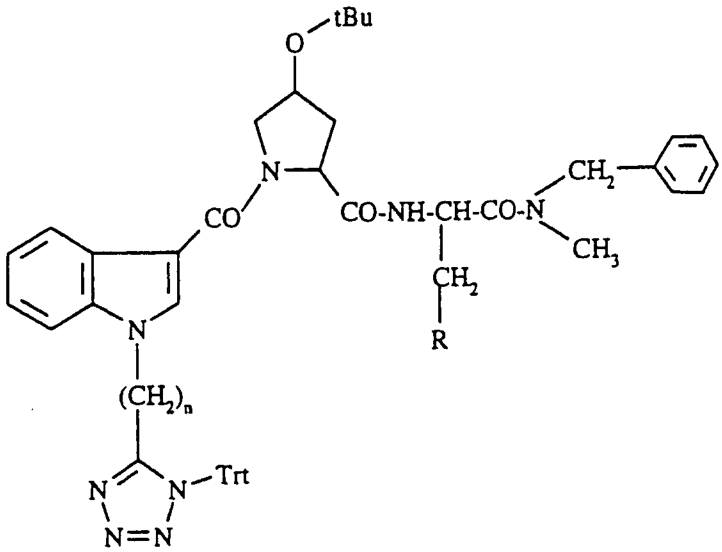

(X)

in der tBu, R und Trt die oben angegebenen Bedeutungen besitzen, von der man die Schutzgruppe in Trifluoressigsäuremedium abspaltet zur Bildung der Verbindung der Formel (I),

- welche gegebenenfalls mit Hilfe einer klassischen Reinigungsmethode gereinigt werden kann,
- welche gegebenenfalls mit Hilfe klassischer Trennmethoden in die Isomeren aufgetrennt werden kann und
- welche man gewünschtenfalls in ihre Additionssalze mit einer pharmazeutisch annehmbaren Base überführt.

**7.** Pharmazeutische Zubereitungen enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien oder Bindemitteln.

**8.** Pharmazeutische Zubereitungen nach Anspruch 7 enthaltend mindestens einen Wirkstoff nach einem der Ansprüche 1 bis 5 zur Verwendung als Antagonisten der Substanz P bei der Behandlung von Schmerzen, Entzündungen, Magen-Darm- oder Harntrakt-Störungen, Asthma, Allergien, Migräne und Erkrankungen des Zentralnervensystems.